(19)

Europäisches Patentamt
European Patent Office
Office européen des brevets

(11) **EP 2 626 147 A1**

(12) **EUROPEAN PATENT APPLICATION**
published in accordance with Art. 153(4) EPC

(43) Date of publication:
**14.08.2013 Bulletin 2013/33**

(21) Application number: **11864619.9**

(22) Date of filing: **10.08.2011**

(51) Int Cl.:
**B08B 3/12** (2006.01)     **A61L 2/025** (2006.01)
**A23L 3/30** (2006.01)     **B01F 11/02** (2006.01)

(86) International application number:
**PCT/RU2011/000602**

(87) International publication number:
**WO 2012/150874 (08.11.2012 Gazette 2012/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.05.2011 RU 2011117049**

(71) Applicant: **Getalov, Andrey Aleksandrovich Moscow 109548 (RU)**

(72) Inventor: **Getalov, Andrey Aleksandrovich Moscow 109548 (RU)**

(74) Representative: **Benatov, Emil Gabriel Dr. Emil Benatov & Partners Asen Peykov Str. No. 6 1113 Sofia (BG)**

(54) **PROCESS FOR THE ULTRASONIC CAVITATION TREATMENT OF LIQUID MEDIA AND OBJECTS ARRANGED IN A MEDIUM**

(57)     The invention relates to the field of cavitation treatment of liquid media as well as media, where the density of water or other liquid phase is over than 65-70% of the total weight as well as to treatment of the objects placed in the treated liquid media. The method cavitation treatment consists is that the vibration system with the liquid medium and the placed objects consists of the wall-surfaces, each surface of the system is the membrane fixed outline, for example on a rigid frame, having a natural frequency with due consideration of the apparent mass of the vibration generator equal to the fundamental mode, the radiation of ultrasonic waves to the liquid media is carried out simultaneously by all membranes of the vibration system, providing in the treated media the effect of superposition of waves with forming a standing acoustic wave or multiple waves with different frequencies; the amplitude of resonant vibrations of each membrane is above the threshold of acoustic cavitation for the liquid media with objects located therein, the vibrations frequencies and phase characteristics of the membranes are chosen so that they can be the same or different to each other in order to maximize the desired cavitation effect factoring in the characteristics of the treated media, at that the vibration system can be of any shape, flowing or steady state mode of motion of the liquid media. The method takes an opportunity to improve the efficiency (power and amplitude of the acoustic wave, coherence) of cavitation effect on the treated liquid media and the objects placed therein while limiting the power of ultrasonic radiators.

EP 2 626 147 A1

**Description**

The invention relates to the field of cavitation treatment of liquid media as well as media, where the density of water or other liquid phase is over than 65-70% of the total weight as well as to treatment of the objects placed in the treated liquid media.

It is known that acoustic ultrasonic cavitation can be effectively applied in different fields of the economy, where the following technology processes are implemented /1-6 /:

- Dispersion;
- Homogenization and emulsification;
- Mixture;
- Disintegration;
- Deagglomeration.

In practice, it covers the processes of production of multicomponent media (emulsions, suspensions, water solutions and water systems), ultrasonic sterilization (disinfection) of water, milk and other products, cleaning tools and medical supplies, etc.

A method of treatment of liquid media, which is implemented in the scheme of the ultrasonic reactor can be taken as a prototype / 1 /. It consists in that the ultrasonic wave in the liquid volume is generated by a rod radiator, at the edge of which the source of vibration is located being usually piezoelectric radiator.

There are many options for calculating the configuration of the rod and the possibility of mounting of several piezo radiators on its edge, but they are all aimed to increase the vibrations amplitude of the rod on the butt and on the sidewalls /8/.

This is due to the fact that in practice the zone of fully-developed cavitation is measured by dimensions of a few centimeters from the vibrations surface. Therefore, the bottom part of the rod is considered the most effective zone, as between the flat edge of the radiator and a flat bottom a standing wave is formed in the treated liquid. At that it is noted that the diameter of the edge is difficult to make more than 50-70 mm.

The radiation from the cylindrical surface of the rod has much smaller amplitude of vibrations and cylindrical divergence. Given the reflected acoustic waves from the walls of the outer cylinder-glass it can be estimated that it is not practically possible to obtain the optimal mode of stable standing flat coherent ultrasonic wave in the treated liquid media, similar to a small area between the edge of the radiator and the bottom of the cylinder-glass.

Complex pattern of transmitted and reflected ultrasonic waves in the medium and the lack of coherence of the wave and the concentration of power at the same frequency leads to the fact that it is not practically possible to obtain an emulsion with the size of the dispersed phase at least 0.8-1.0 m, the level of homogeneity does not exceed 20% for the fundamental mode .At that the volume of treated water is limited.

Another alternative method of ultrasonic cavitation treatment of liquid media is implemented in a rotor-pulsation homogenizers /2/.

In the insonation chamber, due to occurrence of alternative fluid motions taking place regularly from a rotating rotor-stator system, there is an ultrasonic wave with cavitation effects. This is an interim option between the acoustic and hydrodynamic cavitation. Such homogenizers are currently most common. They are uncomplicated enough, take an opportunity to handle large volumes of liquid, they are much cheaper than ultrasonic counterparts. Satisfactory high-velocity homogenizers take opportunity to manufacture an emulsion with the following size of the dispersed phase:- 1.5 mm at the principal mode, the level of homogeneity does not exceed 12-15%. Nevertheless, this method also has some fundamental limitations.

This is due to the poor efficiency of electromechanical systems (down to 10%) that limits the power of ultrasonic waves to 1.5-2 W/cm$^2$ and does not allow treating the viscous media, handling static fluid volumes (in volume stator-rotor) and also has a number of other fundamental limitations.

The nearest equivalent method is the method of obtaining of emulsion cosmetic preparation according to Application No. 2010137176 of September 08, 2010, the positive decision of ROSPATENT of March 22, 2011, No. 2010137176/15 (052870).

Brightening of vibrational amplitude of acoustic wave in the treated liquid medium is effected by resonance in-phase vibrations of each bigger side of channel system having rectangular cross-section and additional superposition of waves inside the channel, at that inside distance is equal to the small side of channel and is multiple of quarter of acoustic wave length in the treated mediume. It takes the opportunity to centralize maximum energy on resonant vibrational frequency of the bigger side of the channel and obtain a standing acoustic wave of high intensity inside the channel.

The research carried out by the company "DERMANIKA" indicated that dominant mode of dispercity in such process of treatment can be - 500 nanometers and less, the emulsion does not practically include dispersion phase with the dimensions more than 1000 nm (1 micron), the proportion of emulsifier in the emulsion is twice or thrice less than usual. At that rotor-pulsing homogenizers take an opportunity to obtain emulsions with the dimensions of disperse phase beginning from 1000 nm (1 micron) and more with the more proportion of emulsifier /2/.

These research was fragmentary reported at the XIV International Research and Practice conference "Cosmetic preparations and raw materials: safety and efficiency" hold on in October 2009, where it was taken second place and the diploma, there are also publications in specialized magazines /6/.

In such a case the quality of products upgrades in accordance with cavitation criteria (cavitation threshold) [3, 4] and resonant mode of operation with the maximum efficiency and the best key figures on intensification of integrated physical-chemical, hydromechanical, heat-exchanging and mass-exchanging processes to the treated medium and the minimum size and homogeneity of oil phase (fat phase) recovered in the output.

This technology is implemented in commercial size in the acting cosmetic manufacturer "Closed Joint Stock Company Laboratory EMANSI". Initial products produced according to this technological process is the hand cream Anti Smell Smoke (for smokers, against influence of nicotine and smoke to hand skin) passed the total cycle of certification tests (Protocol of sanitary and healthcare inspection №77.01.12.915.P.006156.02.10 of February 03, 2010) and и statement of compliance confirmed by independent trails in laboratory "Spectrum" (accreditation certificate № ROSS RU.0001.21PSH50) with the corresponding test sheet No. 19 of December 22, 2009.

However this technology has a number of limitations on use (for example, if it is used for treatment of items put into liquid medium, where acoustic waves are generated). In practice the gap width between the walls of the channel, provided it is required to obtain high intensity, should not be more than half-wavelength. In case the medium is water, it corresponds with the dimension -3,4 cm for the frequency 22 kHz. Besides, it has been noted at various times that cavitation effects amplify in case the liquid is treated on two various frequencies.

In the project /7, page 60/ it is indicated that "in the process of simultaneous impact of ultrasonic waves of two different frequencies (22-44 kHz) it can be seen significant amplification of cavitation efficiency, that is much more stronger than the obtained while line summing up of the impact of each field of different vertical frequency".

In the trials the author also got practical results and the main dependencies of two frequencies influence to obtaining various emulsions (cosmetic emulsions, mayonnaise).

The object of the invention is to increase the efficiency (power and amplitude of the acoustic wave, coherence) of cavitation effects on the treated liquid medium and to the objects placed therein while simultaneous limiting the power of ultrasonic radiators.

This object is achieved due to the fact that the vibrational system with the liquid medium and the object consists of wall-surfaces, each surface of the system is a membrane fixed on a path, for example, on a rigid frame, having the self-resonant frequency with due consideration of the apparent mass of the vibration generator equal to the fundamental harmonic; the emission of ultrasonic waves in a liquid medium is carried out simultaneously by all membranes of vibration system, providing in the treated volume the effect of superposition of waves with forming a standing acoustic wave or multiple waves with different frequencies; the amplitude of resonant vibrations of each membrane is above the threshold of acoustic cavitation for liquid medium with objects placed therein, the vibration frequencies and phase response of the vibrations of membrane are chosen so that they can be the same or different to each other in order to maximize the desired cavitation effect factored in the characteristics of the treated media, with the oscillating system can be of any shape, flow or steady state mode of motion of the liquid medium.

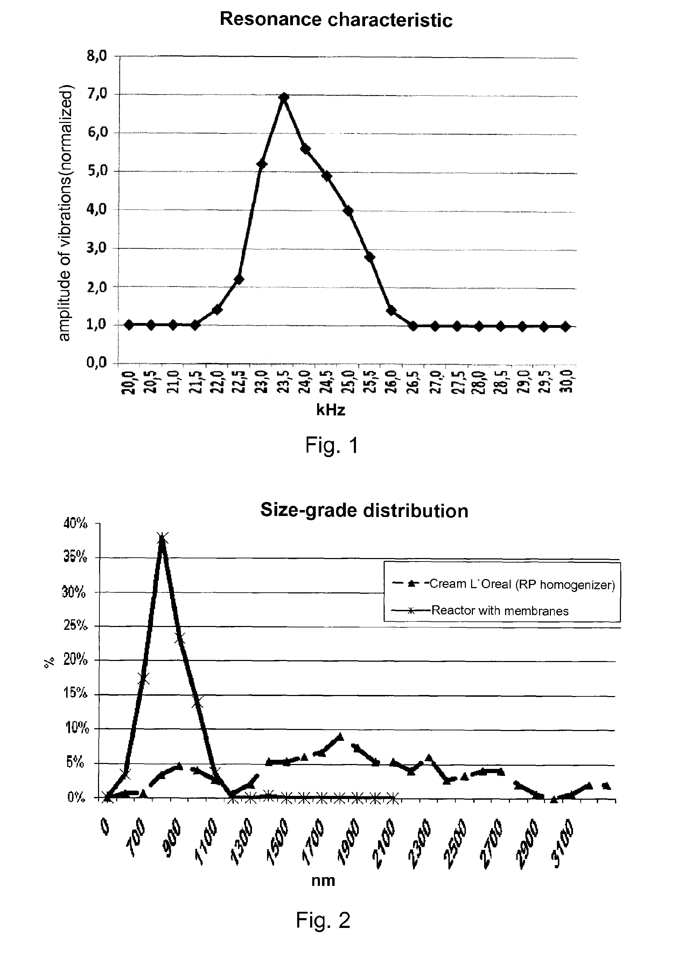

The essence of the invention is explained by drawings, where on a fig. 1 it is represented standard resonant characteristic of a membrane; on a fig. 2 - dependence of the sizes of a disperse phase.

In the proposed method there is used the principle of sequential resonant amplification of acoustic waves at a given frequency, or the number of waves in a given frequencies.

The first vibrations amplitude amplification stage is the resonant characteristic of the membrane whose vibrations are excited by an external source, such as piezo-radiator.

It is known that membranes, in contrast to plates, do not have the flexural rigidity and have higher natural frequencies. The oscillation frequency of a membrane is independent of the thickness, in contrast to the plates. The specific operation mode of a membrane-plate depends on several factors such as the conditions of fixing on the edges (stretch), deflection, periodicity etc. /11/.

For a rectangular membrane with fixed edges the solution of the wave equation on a set of natural frequencies in a Cartesian coordinate system has the form /9, 10/:

$$\omega = c\sqrt{k_x^2 + k_y^2} = c\sqrt{\left(j_x \frac{\pi}{L_x}\right)^2 + \left(j_y \frac{\pi}{L_y}\right)^2}$$

where c - speed of wave propagation on the plate;

kx, ky - wave number, the values of are determined by the boundary conditions;

Lx, - length of the side of the plate is directed along the axis Ox;

Ly - length of the side of the plate is directed along the axis Oy;

jx, jy - an integer equal to the number of crests of the wave along the respective sides of the plate.

To get the peak recoil of the membrane it is required to implement the fundamental mode oscillation mode, when the number of the crests of wave is 1 in both axes. In this case, all the points of the membrane vibrate at the same frequency and phase with the maximum deflection at the center of the membrane. The figure Fig. 1 shows a typical resonance characteristic of the membrane having size 250*145 mm, 1.2 mm thick, made of stainless steel AISI 316 (analog 12X10NT) having the wave velocity - 5800 m/s. It can be seen that at the resonant frequency of ∼ 23.2 kHz the Q factor of the vibration system-cascade is - 7. It takes the opportunity to increase significantly the amplitude of the acoustic wave in the liquid being in contact with this surface, capacity thus brought to a piezo radiator makes - 50 W.

The second-stage amplification of characteristics of the acoustic wave is forming of a standing wave in the liquid or in the area of processing object due to superposition of incident and reflected waves from the wall-surfaces.

For example, if the vibration system looks like the square capacity opened on the one hand, it can contain five square radiating surfaces of membranes. For the frequency of 22 kHz the square party, for AISI 316 steel has to make - 185 mm, thus in the center it is possible to create energy streams in tens $W/cm^2$, the volume of processed liquid makes - 6.3 liters for these conditions.

It is possible to make from above a cover membrane and to receive all 6 radiating surfaces. Such designs can be applied to processing of different foodstuff, including meat, fish, a bird, dough, etc.

Amplitude of oscillations of a membrane (its deflection) can be increased at the expense of a choice of its thickness and conditions of fixing (tension) on a contour. Thus it is necessary to estimate criterion of the maximum deflection of a membrane with allowance for weight piezoelectric radiator.

The numerical ANSYS, MATLAB programs and others allow doing it.

Distances between opposite walls membranes it is expedient to choose multiple quarters of wavelength for the liquid medium.

In Figure 2 it is represented the linear connection of the dispersion phase dimensions for a cosmetic emulsion, obtained on the similar reactor. For comparison the sizes of a cosmetic emulsion cream (moisturizing day cream) of the L'Oreal company are presented which is received on a high-speed high-quality rotor pulsation homogenizer. In this case record level of homogeneity which in the range of 600-700 nm reached - 40% was received.

The implementation of this method at the place of production of DERMANIKA company took the opportunity significantly to increase the efficiency of cavitation effect, to obtain cosmetic emulsion of high quality, to increase the volume of treated liquid - 2-2,5 fold, at that the power of ultrasonic generators was reduced from 6 kW to - 3 kW.

CITED LITERATURE

1. Bronin F.A. Analysis of cavitation fracture and dispergating of solids in high intensity ultrasonic field. Author's abstract thesis in Engineering Science, MISIS, 1967.

2. Chervyakov V.M., Odnolko V.G. Applying of hydrodynamic and cavitation effects in rotor apparatus.- M.: Izd-vo Mashinistrienye, 2008.

3. Sirityuk M.G. Experimental investigations of ultrasonic cavitation. In the book. Intense ultrasonic field, under the editorship of L.D. Rosenberg , 1968.

4. Krasylnikov V.A.Acoustic and ultrasonic waves in the air, water and solids-M.: Fizmatgiz, 1960.

5. Bergman L. Ultrasonics and its application in science and technique.-M.: Inostrannaya literatura, 1956.

6. V.I. Demenko, A.A. Getalov, T.V. Puchkova, E.A. Hotenkova. Effective method impoverishment of emulsifier while production of cosmetic emulsion, magazine "Raw materials and packaging" № 10(101), p.12.

7. Margulis M.A. Fundamental principles of sonochemistry. Chemical reactions in acoustic fields. - M.: Vyshaya Shkola, 1984.

8. Hmelev V.N., Popova O.V. Multifunctional ultrasonic apparatus and their implementation in small productions, agriculture and household conditions; scientific monograph, Alt. Gos. Tekh. Un-t im. I.I. Polzunov.-Bamaul: Izd-vo AltGTU.

9. Koshlyakov N.S., Gliner E.B., Smirnov M.M. Partial equations in mathematical physics. M., Izd-vo Vyshaya shkola, 1970.

10. Armanovich I.G., Levin V.I. Equations of mathematical physics. Second edition, M., Nauka, 1969.

11. Vibrations in technique. Manual in 6 parts, edited by Chalomey V.N., M., Mashinostroenie, 1979.

**EP 2 626 147 A1**

**Claims**

1. Method of ultrasonic cavitation treatment of liquid media and objects located therein by placing them inside a mechanical vibration system, where the regime of acoustic cavitation is implemented due to resonant vibrations of the walls of the channel of rectangular cross section and the standing waves interference in the treated medium, **characterized in that** the vibration system with a liquid medium and placed objects can be of any shape, flowing or steady state mode of motion of the liquid media, at that each surface of the system is the membrane fixed outline, for example on a rigid frame, having a natural frequency with due consideration of the apparent mass of the vibration generator equal to the fundamental mode, the radiation of vibration system, providing in the treated media the effect of superposition of waves with forming a standing acoustic wave or multiple waves with different frequencies; the amplitude of resonant vibrations of each membrane is above the threshold of acoustic cavitation for the liquid media with objects located therein, the vibration frequencies and phase characteristics of the membranes are chosen so that they can be the same or different to each other in order to maximize the desired cavitation effect factoring in the characteristics of the treated media

## Resonance characteristic

Fig. 1

## Size-grade distribution

Fig. 2

## INTERNATIONAL SEARCH REPORT

| International application No. |
|---|
| PCT/RU 2011/000602 |

**A. CLASSIFICATION OF SUBJECT MATTER**
B08B 3/12 (2006.01)  A61L 2/025 (2006.01)  A23L 3/30 (2006.01) B01F 11/02 (2006.01)

According to International Patent Classification (IPC) or to both national classification and IPC

**B. FIELDS SEARCHED**

Minimum documentation searched (classification system followed by classification symbols)
B08B 3/12, A61L 2/025, A23L 3/30, B01F 3/10, 11/02

Documentation searched other than minimum documentation to the extent that such documents are included in the fields searched

Electronic data base consulted during the international search (name of data base and, where practicable, search terms used)

PAJ, Esp@cenet, DWPI, PCT Online, USPTO DB, CIPO, SIPO DB, PatSearch

**C. DOCUMENTS CONSIDERED TO BE RELEVANT**

| Category* | Citation of document, with indication, where appropriate, of the relevant passages | Relevant to claim No. |
|---|---|---|
| A | RU 2009138018 A (GETALOV ANDREI ALEKSANDROVICH) 20.04.2011, the abstract | 1 |
| A | RU 2246347 C1 (OBSCHESTVO S OGRANICHENNOI OTVETSTVENNOSTJU "ASTOR-S") 20.02.2005, the abstract | 1 |
| A | RU 2357810 C2 (OAO "OSOBOE KONSTRUKTORSKOE-TEKHNOLOGICHESKOE BJURO KRISTALL") 10.06.2009, the abstract | 1 |
| A | EA 200601706 A1 (DUDKO MIKHAIL PETROVICH et al.) 28.12.2007 | 1 |
| A | WO 2009/122340 A1 (KONINKLIJKE PHILIPS ELECTRONICS N.V.) 08.10.2009 | 1 |

☐ Further documents are listed in the continuation of Box C.  ☐ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 12 December 2011 (12.12.2011) | 19 January 2012 (19.01.2012) |

| Name and mailing address of the ISA/ | Authorized officer |
|---|---|
| | |
| Facsimile No. | Telephone No. |

Form PCT/ISA/210 (second sheet) (July 1998)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010137176 A

- WO 201013717615 A

**Non-patent literature cited in the description**

- **BRONIN F.A.** Analysis of cavitation fracture and dispergating of solids in high intensity ultrasonic field. *thesis in Engineering Science,* 1967
- **CHERVYAKOV V.M. ; ODNOLKO V.G.** Applying of hydrodynamic and cavitation effects in rotor apparatus.- M. *Izd-vo Mashinistrienye,* 2008
- Experimental investigations of ultrasonic cavitation. In the book. **SIRITYUK M.G.** Intense ultrasonic. editorship of L.D,
- **KRASYLNIKOV V.A.** Acoustic and ultrasonic waves in the air, water and solids-M. *Fizmatgiz,* 1960
- **BERGMAN L.** Ultrasonics and its application in science and technique.-M. *Inostrannaya literatura,* 1956
- **V.I. DEMENKO ; A.A. GETALOV ; T.V. PUCHKOVA ; E.A. HOTENKOVA.** Effective method impoverishment of emulsifier while production of cosmetic emulsion, magazine. *Raw materials and packaging,* 12

- **MARGULIS M.A.** Fundamental principles of sonochemistry. Chemical reactions in acoustic fields. - M. *Vyshaya Shkola,* 1984
- **HMELEV V.N ; POPOVA O.V.** Multifunctional ultrasonic apparatus and their implementation in small productions, agriculture and household conditions; scientific monograph. *Polzunov.-Bamaul: Izd-vo Alt-GTU*
- **KOSHLYAKOV N.S. ; GLINER E.B. ; SMIRNOV M.M.** Partial equations in mathematical physics. M. *Izd-vo Vyshaya shkola,* 1970
- **ARMANOVICH I.G ; LEVIN V.I.** Equations of mathematical physics. 1969
- Vibrations in technique. Manual. 1979